# FASCICULE DE BREVET EUROPEEN

(11) **EP 2 763 632 B1**
(45) Date de publication et mention de la délivrance du brevet: **06.04.2016**
(21) Numéro de dépôt: 12773011.7
(22) Date de dépôt: 31.08.2012
(51) Int. Cl.: A61F 5/56, A61C 7/36

(54) **ORTHÈSE INTRABUCCALE, PROCÉDÉ DE FABRICATION, ET PROCÉDÉ DE REGLAGE D'UNE TELLE ORTHÈSE.**
INTRAORALE ORTHESE SOWIE VERFAHREN ZUR HERSTELLUNG UND VERFAHREN ZUR EINSTELLUNG EINER SOLCHEN ORTHESE
INTRAORAL ORTHOSIS, AND METHOD OF PRODUCING AND METHOD OF ADJUSTING SUCH AN ORTHOSIS

(30) Priorité: 04.10.2011 FR 1158946
(43) Date de publication de la demande: 13.08.2014
(73) Titulaire: BLUESOM, 44700 Orvault (FR)
(72) Inventeur: PÉTELLE, Boris, 75009 Paris (FR); FLEURY, Bernard, 94300 Vincennes (FR)
(74) Mandataire: CAPRI
(86) Numéro de dépôt international: PCT/FR2012/051963
(87) Numéro de publication internationale: WO 2013/050679

(56) Documents cités:
- WO-A1-2008/023799
- US-A- 5 570 704
- US-A1- 2005 081 859
- US-A1- 2010 105 001
- US-A1- 2011 036 357
- US-A1- 2011 100 379
- US-B2- 7 354 270

## Description

La présente invention concerne une orthèse intrabuccale, et plus particulièrement une telle orthèse destinée au traitement du ronflement et du syndrome d'apnées du sommeil. La présente invention concerne aussi un procédé de fabrication et un procédé de réglage d'une telle orthèse intrabuccale.

Les orthèses intrabuccales pour traiter le ronflement et les apnées du sommeil sont connus dans l'état de la technique. Ces orthèses comportent généralement une gouttière maxillaire et une gouttière mandibulaire qui sont reliées par un dispositif de liaison permettant de régler la position relative de la gouttière mandibulaire par rapport à la gouttière maxillaire. Il a été constaté que pour la plupart des personnes, il existe une position de la mâchoire dans laquelle les ronflements et les apnées diminuent voire disparaissent. Cette position est différente selon les utilisateurs, et il est donc nécessaire de déterminer avec précision la bonne position pour pouvoir régler l'orthèse pour chaque utilisateur. Le document US 7,354,270 décrit une orthèse intrabuccale selon le préambule de la revendication 1 annexée.

Les documents WO 97/04716, US 5,409,017, US 5,820,579, WO 02/38090, WO 2006/013238, US 2011/0036357, US 7,354,270, US 2005/0081859 et WO 2008/023799 décrivent d'autres dispositifs de l'art antérieur. Ces dispositifs présentent un certain nombre d'inconvénients. Ainsi, l'adaptation et le réglage des gouttières et de leur position sont généralement assez complexes et doivent être réalisée par un spécialiste. Ceci impose de nombreuses visites et présente donc un coût important. De plus, pour que l'orthèse soit parfaitement adaptée à chaque utilisateur, il faut généralement réaliser des moulages des dents de l'utilisateur pour ensuite fabriquer les gouttières sur mesure en laboratoire en fonction de ces moulages. Les qualités requises pour une orthèse sont en particulier un volume réduit pour limiter l'inconfort et une rétention satisfaisante des gouttières sur les arcades dentaires notamment pour être certain de l'absence de désadaptation nocturne source d'une perte d'efficacité. De plus, il est souhaitable d'avoir un réglage possible du système de liaison des gouttières qui soit fiable et précis, en particulier millimétrique, afin de permettre une titration, à savoir un réglage précis de la position optimale de l'orthèse. Par ailleurs, le réglage doit être verrouillable pour éviter tout déréglage, notamment pendant le sommeil.

A ce jour, il n'existe pas d'orthèse préfabriquée dont le volume, la qualité de rétention et le réglage soit comparable aux dispositifs sur mesure fabriqués en laboratoire.

La présente invention a pour but de fournir une orthèse intrabuccale qui ne reproduit pas les inconvénients susmentionnés.

En particulier, la présente invention a pour but de fournir une orthèse intrabuccale qui soit simple, réglable précisément, peu volumineuse, confortable, rétentive, comparable à une orthèse sur mesure mais peu coûteuse à fabriquer et à utiliser.

Plus particulièrement, la présente invention a pour but de fournir une orthèse intrabuccale qui peut être aisément adaptée à chaque utilisateur et réglée de façon précise et simplement c'est-à-dire sans instrument particulier.

La présente invention a donc pour objet une orthèse telle que définie dans la revendication 1 annexée, comportant une première coque et une seconde coque, lesdites coques étant reliées entre elles par un dispositif de liaison réglable, ledit dispositif de liaison réglable comportant deux pattes crantées qui sont respectivement disposées de chaque côté latéral de l'une desdites coques, chaque patte crantée coopérant de manière réglable avec un logement de réglage respectif, disposé de chaque côté de l'autre desdites coques, chaque patte crantée comportant d'une part des dents de réglages pour régler la position des coques l'une par rapport à l'autre, et d'autre part des logements de verrouillage pour verrouiller ladite position, lesdits logements de verrouillage étant orientés transversalement par rapport auxdites dents de réglage.

Avantageusement, chaque logement de réglage comporte au moins une dent de réglage adaptée à coopérer avec au moins une dent de réglage respective de la patte crantée correspondante pour définir une position de la première coque par rapport à la seconde coque.

Avantageusement, ledit dispositif de liaison réglable est adapté à définir la position relative des deux coques entre une position reculée et une position avancée de la coque mandibulaire.

Avantageusement, ledit dispositif de liaison réglable limite l'ouverture buccale lors de l'utilisation de l'orthèse.

Avantageusement, les coques sont réalisées par moulage et/ou pliage.

Avantageusement, les coques sont réalisées en un matériau relativement souple, tel que le polypropylène ou le Rilsan®, permettant d'adapter chaque coque à l'arcade dentaire correspondante.

Avantageusement, les logements de réglage sont moulés d'une pièce monobloc avec leur coque respective, chaque logement de réglage étant relié à ladite coque par une structure de support pliable.

Avantageusement, chaque logement de réglage comporte un système de verrouillage.

Avantageusement, ledit système de verrouillage comporte un élément de verrouillage déplaçable par rapport audit logement de réglage entre une position de verrouillage, dans laquelle des projections de verrouillage de l'élément de verrouillage coopèrent avec lesdits logements de verrouillage de ladite patte crantée, et une position de déverrouillage, dans laquelle lesdites projections de verrouillage ne coopèrent pas avec lesdits logements de verrouillage.

Avantageusement, lesdits projections et logements de verrouillage sont en forme de cannelures complémentaires.

Avantageusement, ledit élément de verrouillage comporte au moins un épaulement coopérant avec ledit logement de réglage en position de déverrouillage, pour empêcher le retrait dudit élément de verrouillage dudit logement de réglage.

Avantageusement, ledit élément de verrouillage comporte au moins un bourrelet coopérant avec ledit logement de réglage lors du déplacement dudit élément de verrouillage entre ses positions de verrouillage et de déverrouillage, pour créer une résistance au déplacement et ainsi empêcher tout déplacement non souhaité.

Avantageusement, chaque coque comporte un élément d'empreinte respectif réalisé en matériau thermoformable pour épouser la forme des dents de l'utilisateur, chaque élément d'empreinte étant fixé à sa coque respective par surmoulage.

Avantageusement, les éléments d'empreinte sont réalisés en un matériau sensiblement rigide à température ambiante et sensiblement déformable lorsqu'il est chauffé.

Avantageusement, les éléments d'empreinte sont réalisés en un matériau choisi dans la famille des Polycapronolactones (PCL), tel que le CAPA®.

Avantageusement, lesdites coques comportent des profils d'accrochage, tels que des nervures, rainures ou projections, pour améliorer la fixation des éléments d'empreinte lors du surmoulage.

La présente invention a aussi pour objet un procédé de fabrication d'une orthèse telle que décrite ci-dessus, comportant les étapes suivantes : moulage des coques maxillaire et mandibulaire, surmoulage des éléments d'empreinte dans lesdites coques, fabrication du dispositif de liaison réglable, notamment par pliage d'au moins une partie d'au moins une coque, assemblage des coques au niveau du dispositif de liaison réglable.

La présente invention a aussi pour objet un procédé d'utilisation d'une orthèse réalisé selon le procédé de fabrication décrit ci-dessus, comprenant les étapes suivantes : chauffage des éléments d'empreinte à une température d'utilisation dans laquelle les éléments d'empreinte sont déformables, mise en place de l'orthèse avec ses éléments d'empreinte chauffés dans la bouche de l'utilisateur pour former des gouttières en moulant les éléments d'empreinte sur les dents de l'utilisateur, refroidissement desdits éléments d'empreinte, réglage de la position relative des coques maxillaire et mandibulaire au moyen du dispositif de liaison réglable.

Ces caractéristiques et avantages et d'autres de la présente invention apparaitront plus clairement au cours de la description détaillée suivante d'un mode de réalisation avantageux de celle-ci, faite en référence aux dessins joints, donnés à titre d'exemple non limitatifs, et sur lesquels
La figure 1 est une vue en section transversale de derrière d'une orthèse selon un mode de réalisation avantageux de la présente invention, dans une position où les deux coques sont superposées,
La figure 2 est une vue en section transversale de devant de l'orthèse de la figure 1, dans ladite position superposée,
La figure 3 est une vue en section transversale de côté de l'orthèse des figures 1 et 2, toujours en position superposée,
La figure 4 est une vue similaire à celle de la figure 3, dans une position avancée de la coque mandibulaire,
La figure 5 est une vue de dessus de l'orthèse de la figure 4,
La figure 6 est une vue de détail d'une coque maxillaire,
La figure 7 est une vue de détail d'une coque mandibulaire,
La figure 8 est une vue schématique en perspective d'une orthèse avec les deux coques assemblées, selon une variante avantageuse de l'invention,
La figure 9 est une vue de détail du système de réglage de l'orthèse de la figure 8,
Les figures 10 et 11 sont des vues de détail du système de verrouillage de l'orthèse de la figure 8, respectivement en position de verrouillage et de déverrouillage, et
La figure 12 est une vue schématique d'une variante du système de verrouillage, en position de verrouillage.

En référence aux dessins, l'orthèse selon la présente invention comporte une première coque 10 et une seconde coque 20. L'une des deux coque est la coque maxillaire et l'autre coque est la coque mandibulaire. Sur les figures, la coque maxillaire 10 est représentée comme étant la coque supérieure et la coque mandibulaire 20 est représentée comme étant la coque inférieure. Chaque coque présente une forme arrondie et creuse et est réalisée en un matériau relativement souple, tel que du polypropylène ou du Rilsan®, pour permettre d'adapter les coques sur toutes les tailles d'arcades dentaires. Ces coques sont avantageusement réalisées principalement par moulage.

Les deux coques sont reliées l'une à l'autre par un dispositif de liaison réglable 30.

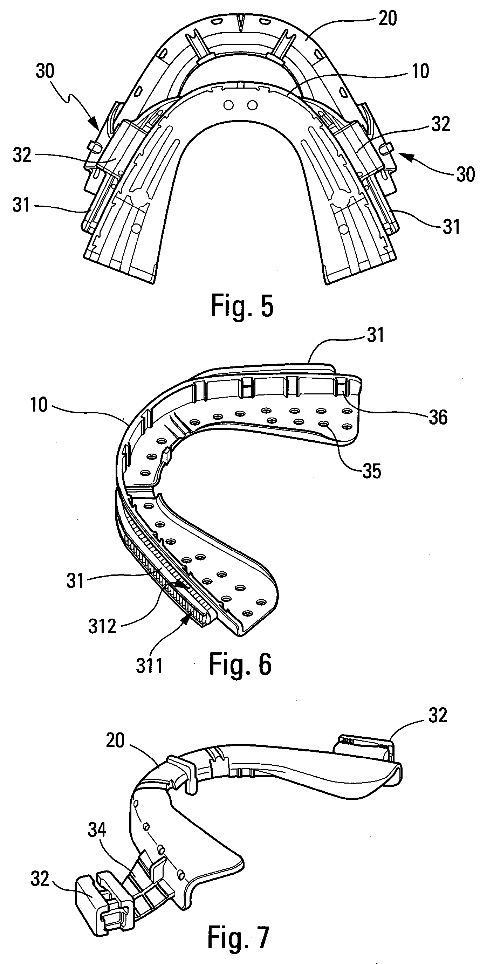

Comme visible sur les figures, ce dispositif de liaison comporte deux pattes crantées 31, avec une patte crantée 31 disposée de chaque côté d'une coque de l'orthèse, par exemple la coque maxillaire 10 comme représenté sur la figure 6. L'autre coque, dans ce cas la coque mandibulaire 20, comporte alors deux logements de réglage 32 recevant chacun une patte crantée 31 pour définir avec précision la position de la coque mandibulaire 20 par rapport à la coque maxillaire 10.

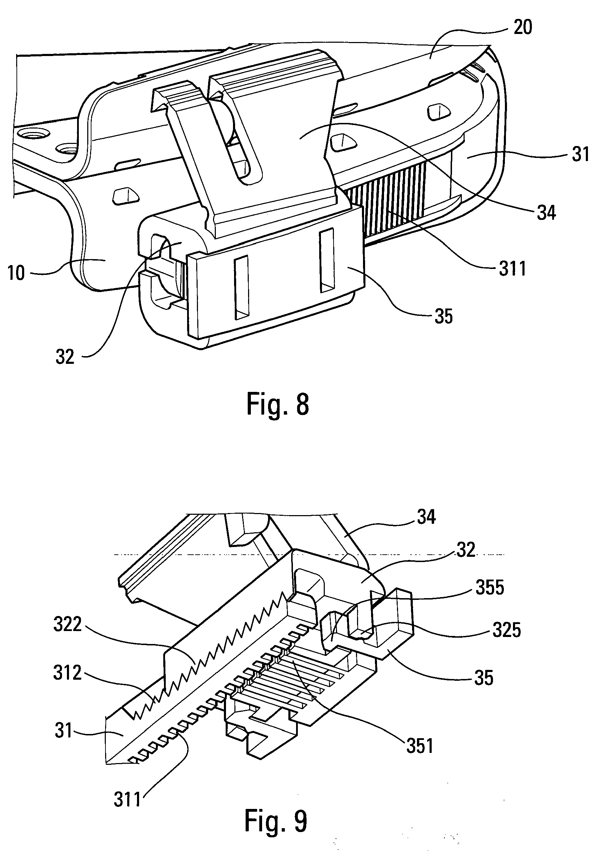

Bien entendu, le dispositif de liaison réglable pourrait être inversé, c'est-à-dire avec les pattes crantées disposées sur la coque mandibulaire et les logements de réception sur la coque maxillaire, comme représenté par exemple sur la figure 8.

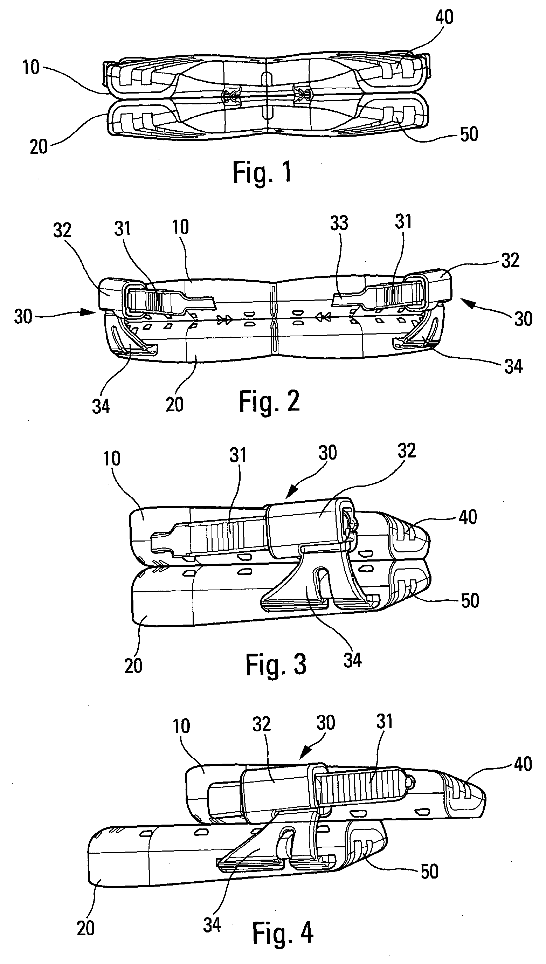

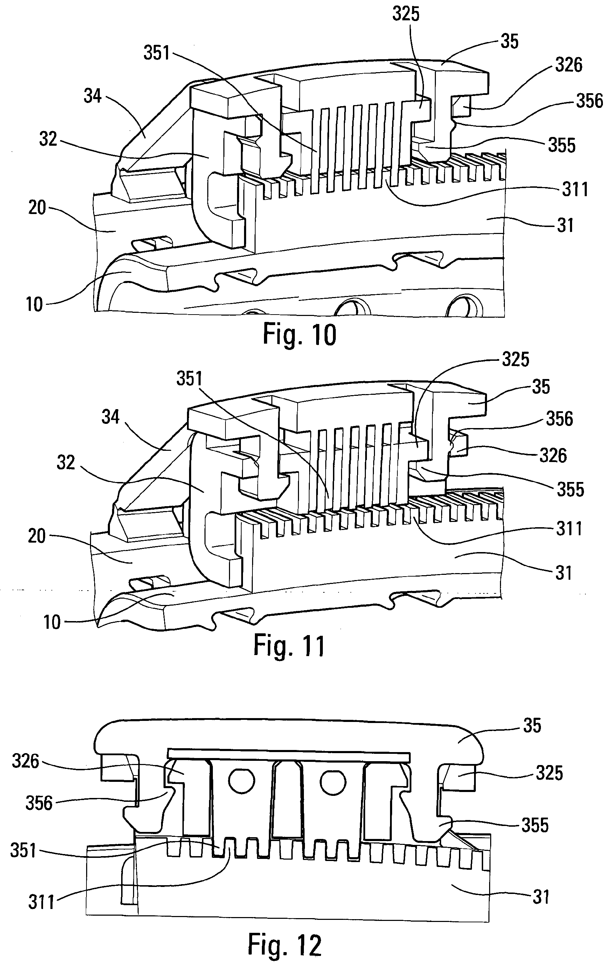

Comme visible sur la figure 10, chaque logement de réglage 32 comporte au moins une dent de réglage 322, en l'occurrence sur cet exemple une pluralité de dents de réglage 322, qui viennent coopérer avec au moins une dent de réglage 312, en l'occurrence sur cet exemple une pluralité de dents de réglage 312, des pattes crantées 31 pour régler avec précision la position de l'orthèse. De préférence, ces dents de réglage 312 et 322 sont de formes complémentaires, par exemple pointues, comme visible sur la figure 10. De préférence, les dents 312 des pattes crantées 31 sont orientées axialement, c'est à dire verticalement dans la position de l'orthèse représentée sur les figures 1 à 4 et 8. Il est à noter que le nombre, la forme et l'orientation des dents de réglage 312 et 322 peuvent être quelconques.

La seconde coque 20 représentée sur la figure 7 comporte deux logements de réglage 32 qui sont reliés à la seconde coque 20 par une structure de support 34 respective pliable. Cette seconde coque 20 avec ses structures de support 34, et ses logements de réglage 32 peut ainsi être réalisée par moulage et pliage. Le procédé de fabrication de l'orthèse est donc particulièrement simple et ne nécessite aucune manipulation complexe.

Lorsque le logement de réglage 32 est replié en position d'utilisation, il suffit pour assembler la coque mandibulaire à la coque maxillaire d'insérer les pattes crantées 31 dans lesdits logements 32 et de régler la position de l'orthèse avec précision en faisant avancer et/ou reculer lesdites pattes crantées dans lesdits logements de réglage 32. Le dispositif de liaison réglable 30 fonctionne donc avantageusement à la manière d'un collier de serrage utilisé par exemple pour fixer des câbles.

Un système de verrouillage 35 permet avantageusement de figer la position retenue comme efficace pour chaque patient. Les figures 9 à 12 illustrent un mode de réalisation avantageux de ce système de verrouillage. Dans cet exemple, chaque logement de réglage 32 comporte un élément de verrouillage 35 qui comporte au moins une, de préférences plusieurs projections de verrouillage 351, adaptées à coopérer avec des logements de verrouillage 311 prévus sur chaque patte crantée 31. De préférence, ces logements de verrouillage 311 sont formés par des cannelures orientées transversalement, notamment perpendiculairement, par rapport à l'orientation axiale des dents 311. Comme visible sur les figures 9 à 12, les projections et logements de verrouillage ont des formes de cannelures complémentaires. L'élément de verrouillage 35 est déplaçable par rapport à son logement de réglage respectif 32 entre une position de verrouillage, visible sur les figures 10 et 12, et une position de déverrouillage, visible sur les figures 9 et 11. Avantageusement, l'élément de verrouillage 35 comporte un épaulement 355 coopérant avec une butée 325 dans la position de déverrouillage. Ceci empêche un démontage accidentel de l'élément de verrouillage 35 de son logement de réglage 32. Avantageusement, l'élément de verrouillage 35 comporte aussi un bourrelet 356 coopérant avec une bride 326 du logement de réglage 32, ledit bourrelet étant en-dessous de ladite bride en position de verrouillage et au-dessus de celle-ci en position de déverrouillage. Ceci permet de créer une résistance au déplacement de l'élément de verrouillage 35, pour éviter tout déverrouillage non souhaité.

Dans la variante des figures 10 et 11, l'élément de verrouillage 35 comporte deux épaulements 355 tournés vers l'intérieur, et deux bourrelets 356 tournés vers l'extérieur. Dans la variante de la figure 12, il y a deux épaulements 355 tournés vers l'extérieur et deux bourrelets 356 tournés vers l'intérieur. De manière générale, le nombre, la forme et l'orientation des épaulements 355 et des bourrelets 356 peuvent être quelconques.

La figure 6 illustre un mode de réalisation préféré des pattes crantées 31. Dans cette variante, chaque patte crantée 31 est formée par une partie de tige s'étendant le long d'un coté de la coque 10, ladite partie de tige comportant les logements de verrouillage 311 sur sa face latérale externe. Cette partie de tige est reliée à ladite coque 10 par un pont de matière de dimensions réduite, de sorte qu'en section transversale, ladite patte crantée a environ la forme d'un T s'étendant latéralement à partier de la coque 10. Ce pont de matière comporte sur sa surface supérieure (dans la position de la figure 6) ou sur sa surface inférieure, des dents de réglages 312. Celles-ci s'étendent donc dans une direction environ perpendiculaire aux logements de verrouillage 311.

La figure 7 illustre un mode de réalisation préféré des logements de réglage 32 adaptés à coopérer avec les pattes crantées de la figure 6. Dans cette variante, chaque logement de réglage comporte un boitier creux dans lequel peut coulisser une patte crantée. Ce boitier creux définit une fente longitudinale dans laquelle passe le pont de matière de la patte crantée, avec un bord latéral de ladite fente qui comporte les dents de réglage 322 adaptées à coopérer avec les dents de réglage 311. La face externe du boitier creux comporte des ouvertures permettant l'assemblage de l'élément de verrouillage 35.

Les figures 3 et 4 illustrent les deux positions d'extrémités du réglage, avec dans la figure 3 les coques qui sont superposées, correspondant à la position reculée de la coque mandibulaire, et dans la figure 4, la coque mandibulaire en position avancée.

L'invention permet ainsi un réglage précis, en particulier millimétrique, de l'orthèse sans instrument, avec si nécessaire la possibilité d'un réglage asymétrique sur les deux cotés. Le réglage est modifiable à volonté sans dégradation du système, le système de verrouillage évitant toute modification de position spontanée et/ou non souhaitée.

Avantageusement, le dispositif de liaison réglable 30 exerce une contrainte qui limite l'ouverture buccale, celle-ci étant connue pour être néfaste pour l'action satisfaisante du dispositif de propulsion mandibulaire. Ainsi, ceci améliore encore l'efficacité de l'orthèse en utilisation.

Le dispositif de liaison réglable 30 possède une articulation liée au pliage des structures de support 34, ce qui permet d'accepter des mouvements latéraux limités des coques 10, 20, améliorant le confort sans toutefois autoriser le recul de la coque mandibulaire.

Selon un autre aspect avantageux, l'orthèse selon l'invention comporte dans chaque coque 10, 20 un élément d'empreinte réalisé en matériau thermoformable, c'est-à-dire un matériau qui, lorsqu'il est chauffé, devient déformable. Ce type de matériau, sensiblement rigide à température ambiante et sensiblement déformable lorsqu'il est chauffé, comprend notamment les matériaux de la famille des polycapronolactones (PCL), et notamment le CAPA®. Selon un aspect particulièrement avantageux, ces éléments d'empreinte 40, 50 sont fixées dans leurs coques respectives 10, 20 par surmoulage. Ainsi, aucune couche de collage, de liaison ou autre n'est nécessaire entre l'élément d'empreinte et la coque. La fabrication de l'orthèse en est donc simplifiée.

Pour améliorer la fixation des éléments d'empreinte aux coques, les coques 10, 20 comportent avantageusement des profils d'accrochage 35, 36, tels que par exemple des nervures, des rainures, des projections. La figure 6, qui montre la première coque 10, illustre des exemples de tels profils d'accrochage, et des profils similaires sont avantageusement prévus dans la seconde coque 20. Ces profils d'accrochage permettent de renforcer la fixation des éléments d'empreinte lors de leur surmoulage sur les coques.

L'utilisation d'éléments d'empreinte en polycapronolactone est avantageuse puisqu'elle permet à l'utilisateur de réaliser lui-même le moulage de ses dents après avoir chauffé l'orthèse. Typiquement, la température de chauffage n'excédera pas 50°C, pour éviter tout risque de blessure, et l'utilisateur disposera alors l'orthèse avec les éléments d'empreinte chauffés dans sa bouche pour réaliser le moulage de ses dents, et former ainsi deux gouttières, une pour chaque arcade dentaire. Après refroidissement, ce moulage restera sensiblement non-déformable à température ambiante et assurera donc une parfaite adaptation de l'orthèse à la bouche de l'utilisateur pendant son utilisation. Ainsi, après avoir réalisé le moulage de ses dents, l'utilisateur pourra utiliser l'orthèse en réglant très facilement lui-même la position de la seconde coque 20 par rapport à la première coque 10 au moyen du dispositif de liaison réglable 30.

La présente invention permet donc de fournir une orthèse intrabuccale qui peut être fabriquée facilement et à faible coût et qui peut être proposée à la vente dans le commerce, chaque utilisateur pouvant réaliser aisément l'adaptation de l'orthèse à sa bouche par moulage de ses dents et par réglage de la position de l'orthèse. Aucune manipulation complexe n'est nécessaire pour réaliser cette adaptation et ce réglage. Le dispositif de l'invention est donc utilisable par un grand nombre d'utilisateurs, de par sa simplicité de mise en oeuvre et de son coût réduit. Il est à noter que le système de crantage latéral pourrait être utilisé sur des gouttières réalisées de façon traditionnelle, c'est-à-dire en laboratoire, pour certains patients dont la denture le nécessite.

Bien que la présente invention ait été décrite en référence à divers modes de réalisation avantageux de celle-ci, il est entendu que l'invention n'est pas limitée à ces modes de réalisation, mais qu'au contraire toutes modifications utiles peuvent être apportées par l'homme du métier sans sortir du cadre de la présente invention tel que défini par les revendications annexées.

## Revendications

1. Orthèse intrabuccale comportant une première coque (10) et une seconde coque (20), lesdites coques (10, 20) étant reliées entre elles par un dispositif de liaison réglable (30), ledit dispositif de liaison réglable (30) comportant deux pattes crantées (31) qui sont respectivement disposées de chaque côté latéral de l'une desdites coques (10, 20), chaque patte crantée (31) coopérant de manière réglable avec un logement de réglage (32) respectif, disposé de chaque côté de l'autre desdites coques (20, 10), chaque patte crantée (31) comportant des dents de réglages (312) pour régler la position des coques (10, 20) l'une par rapport à l'autre, chaque logement de réglage (32) comportant au moins une dent de réglage (322) adaptée à coopérer avec au moins une dent de réglage respective (312) de la patte crantée (31) correspondante pour définir une position de la première coque (10) par rapport à la seconde coque (20),
**caractérisée en ce que**
chaque patte crantée (31) comporte des logements de verrouillage (311) pour verrouiller la position des coques (10, 20) l'une par rapport à l'autre, lesdits logements de verrouillage (311) étant orientés transversalement par rapport auxdites dents de réglage (312), et **en ce que** chaque logement de réglage (32) comporte un système de verrouillage (35) comportant un élément de verrouillage (35) déplaçable par rapport audit logement de réglage (32) entre une position de verrouillage, dans laquelle des projections de verrouillage (351) de l'élément de verrouillage (35) coopèrent avec lesdits logements de verrouillage (311) de ladite patte crantée (31), et une position de déverrouillage, dans laquelle lesdites projections de verrouillage (351) ne coopèrent pas avec lesdits logements de verrouillage (311).

2. Orthèse selon la revendication 1, dans laquelle la position relative des deux coques (10, 20) est réglable entre une position reculée et une position avancée de la coque mandibulaire.

3. Orthèse selon l'une quelconque des revendications précédentes, dans laquelle les coques (10, 20) sont réalisées par moulage et/ou pliage.

4. Orthèse selon la revendication 3, dans laquelle les coques (10, 20) sont réalisées en un matériau relativement souple, tel que le polypropylène ou le Rilsan®, permettant d'adapter chaque coque à l'arcade dentaire correspondante.

5. Orthèse selon l'une quelconque des revendications précédentes, dans laquelle les logements de réglage (32) sont moulés d'une pièce monobloc avec leur coque respective (20, 10), chaque logement de réglage (32) étant relié à ladite coque (20, 10) par une structure de support pliable (34).

6. Orthèse selon l'une quelconque des revendications précédentes, dans laquelle lesdits projections et logements de verrouillage (351, 311) sont en forme de cannelures complémentaires.

7. Orthèse selon l'une quelconque des revendications précédentes, dans laquelle ledit élément de verrouillage (35) comporte au moins un épaulement (355) coopérant avec ledit logement de réglage (32) en position de déverrouillage, pour empêcher le retrait dudit élément de verrouillage dudit logement de réglage.

8. Orthèse selon l'une quelconque des revendications précédentes, dans laquelle ledit élément de verrouillage (35) comporte au moins un bourrelet (356) coopérant avec ledit logement de réglage (32) lors du déplacement dudit élément de verrouillage entre ses positions de verrouillage et de déverrouillage, pour créer une résistance au déplacement et ainsi empêcher tout déplacement non souhaité.

9. Orthèse selon l'une quelconque des revendications précédentes, dans laquelle chaque coque (10, 20) comporte un élément d'empreinte respectif (40, 50) réalisé en matériau thermoformable pour épouser la forme des dents de l'utilisateur, chaque élément d'empreinte (40, 50) étant fixé à sa coque respective (10, 20) par surmoulage.

10. Orthèse selon la revendication 9, dans laquelle les éléments d'empreinte (40, 50) sont réalisés en un matériau sensiblement rigide à température ambiante et sensiblement déformable lorsqu'il est chauffé.

11. Orthèse selon la revendication 10, dans laquelle les éléments d'empreinte (40, 50) sont réalisés en un matériau choisi dans la famille des Polycapronolactones (PCL), tel que le CAPA®.

12. Orthèse selon l'une quelconque des revendications 9 à 11, dans laquelle lesdites coques (10, 20) comportent des profils d'accrochage (35, 36), tels que des nervures, rainures ou projections, pour améliorer la fixation des éléments d'empreinte (40, 50) lors du surmoulage.

13. Procédé de fabrication d'une orthèse selon l'une quelconque des revendications précédentes, comportant les étapes suivantes :
moulage des coques maxillaire et mandibulaire (10, 20), surmoulage des éléments d'empreinte (40, 50) dans lesdites coques (10, 20), fabrication du dispositif de liaison réglable (30), notamment par pliage d'au moins une partie d'au moins une coque (10, 20), assemblage des coques (10, 20) au niveau du dispositif de liaison réglable (30).

14. Procédé de réglage d'une orthèse réalisée selon le procédé de fabrication de la revendication 13, comprenant les étapes suivantes :
chauffage des éléments d'empreinte (40, 50) à une température d'utilisation dans laquelle les éléments d'empreinte sont déformables,
mise en place de l'orthèse avec ses éléments d'empreinte (40, 50) chauffés dans la bouche de l'utilisateur pour former des gouttières en moulant les éléments d'empreinte (40, 50) sur les dents de l'utilisateur,
refroidissement desdits éléments d'empreinte (40, 50), réglage de la position relative des coques maxillaire et mandibulaire (10, 20) au moyen du dispositif de liaison réglable (30).

## Patentansprüche

1. Intraorale Orthese mit einer ersten Schale (10) und einer zweiten Schale (20), wobei die Schalen (10, 20) über eine einstellbare Verbindungsvorrichtung (30) miteinander verbunden sind, wobei die einstellbare Verbindungsvorrichtung (30) zwei gerippte Laschen (31) aufweist, die entsprechend auf jeder Seite von einer der Schalen (10, 20) angeordnet sind, wobei jede gerippte Lasche (31) in einstellbarer Art und Weise mit einem jeweiligen Einstellaufnahmeteil (32) zusammenwirkt, das auf jeder Seite der anderen Schale (20, 10) angeordnet ist, wobei jede gerippte Lasche (31) Einstellzähne (312) zum Einstellen der Position der Schalen (10, 20) zueinander aufweist,
wobei jedes Einstellaufnahmeteil (32) zumindest einen Einstellzahn (322) aufweist, der dazu ausgelegt ist, mit zumindest einem jeweiligen Einstellzahn (312) der entsprechenden gerippten Lasche (31) zusammenzuwirken, um eine Position der ersten Schale (10) bezüglich der zweiten Schale (20) zu definieren,
**dadurch gekennzeichnet, dass**
jede gerippte Lasche (31) Verriegelungsaufnahmen (311) zum Verriegeln der Position der Schalen (10, 20) zueinander aufweist, wobei die Verriegelungsaufnahmen (311) quer zu den Einstellzähnen (312) ausgerichtet sind, und dass jedes Einstellaufnahmeteil (32) ein Verriegelungssystem (35) aufweist, das ein Verriegelungselement (35) enthält, das bezüglich des Einstellaufnahmeteils (32) zwischen einer Verrieglungsposition, in welcher Verriegelungsvorsprünge (351) des Verriegelungselements (35) mit den Verriegelungsaufnahmen (311) der gerippten Lasche (31) zusammenwirken, und einer Entriegelungsposition verstellbar ist, in welcher die Verriegelungsvorsprünge (351) nicht mit den Verriegelungsaufnahmen (311) zusammenwirken.

2. Orthese nach Anspruch 1, wobei die relative Position der beiden Schalen (10, 20) zwischen
einer zurückgezogenen Position und einer vorgerückten Position der Unterkieferschale einstellbar ist.

3. Orthese nach einem der vorangehenden Ansprüche, wobei die Schalen (10, 20) durch Formstanzen und/oder Biegen hergestellt sind.

4. Orthese nach Anspruch 3, wobei die Schalen (10, 20) aus einem relativ nachgiebigen Material, wie etwa Polypropylen oder Rilsan®, hergestellt sind, mit dem es möglich ist, jede Schale an den entsprechenden Zahnbogen anzupassen.

5. Orthese nach einem der vorangehenden Ansprüche, wobei die Einstellaufnahmeteile (32) aus einem Teil geformt sind, das einstückig mit deren jeweilige Schale (20, 10) ausgeführt ist, wobei jedes Einstellaufnahmeteil (32) über eine biegbare Haltestruktur (34) mit der Schale (20, 10) verbunden ist.

6. Orthese nach einem der vorangehenden Ansprüche, wobei die Verriegelungsvorsprünge und -aufnahmen (351, 311) in Form von komplementären Rillen ausgebildet sind.

7. Orthese nach einem der vorangehenden Ansprüche, wobei das Verriegelungselement (35) zumindest eine Schulter (355) aufweist, die mit dem Einstellaufnahmeteil (32) in Entriegelungsposition zusammenwirkt, um das Entfernen des Verriegelungselements aus dem Einstellaufnahmeteil zu verhindern.

8. Orthese nach einem der vorangehenden Ansprüche, wobei das Verriegelungselement (35) zumindest eine Wulst (356) aufweist, die mit dem Einstellaufnahmeteil (32) bei der Verlagerung des Verriegelungselements zwischen seiner Verriegelungs- und seiner Entriegelungsposition zusammenwirkt, um einen Verlagerungswiderstand zu schaffen und damit jegliche unerwünschte Verlagerung zu verhindern.

9. Orthese nach einem der vorangehenden Ansprüche, wobei jede Schale (10, 20) ein jeweiliges Abdruckelement (40, 50) aufweist, das aus einem wärmeformbaren Material hergestellt ist, um sich an die Form der Zähne des Benutzers anzuschmiegen, wobei jedes Abdruckelement (40, 50) durch Anformen an seine jeweilige Schale (10, 20) fixiert ist.

10. Orthese nach Anspruch 9, wobei die Abdruckelemente (40, 50) aus einem Material hergestellt sind, das bei Raumtemperatur im Wesentlichen starr und bei Erhitzen im Wesentlichen verformbar ist.

11. Orthese nach Anspruch 10, wobei die Abdruckelemente (40, 50) aus einem Material hergestellt sind, das aus der Familie der Polycapronolactone (PCL), wie etwa CAPA®, ausgewählt ist.

12. Orthese nach einem der Ansprüche 9 bis 11, wobei die Schalen (10, 20) Verhakungsprofile (35, 36), wie etwa Rippen, Rillen oder Vorsprünge, aufweisen, um die Fixierung der Abdruckelemente (40, 50) beim Anformen zu verbessern.

13. Verfahren zum Herstellen einer Orthese nach einem der vorangehenden Ansprüche, umfassend die nachfolgenden Schritte:
Formstanzen der Oberkiefer- und der Unterkieferschale (10, 20), Anformen von Abdruckelementen (40,50) in den Schalen (10, 20), Herstellen der einstellbaren Verbindungsvorrichtung (30), insbesondere durch Biegen von zumindest einem Teil zumindest einer Schale (10, 20), und Zusammenfügen der Schalen (10, 20) im Bereich der einstellbaren Verbindungsvorrichtung (30).

14. Verfahren zum Einstellen einer Orthese, die mit dem Herstellungsverfahren nach Anspruch 13 hergestellt ist, umfassend die nachfolgenden Schritte:
Erhitzen der Abdruckelemente (40, 50) auf eine Gebrauchstemperatur, bei welcher die Abdruckelemente verformbar sind, Einsetzen der Orthese mit ihren erhitzten Abdruckelementen (40, 50) in den Mund des Benutzers, um Rillen zu bilden, indem die Abdruckelemente (40, 50) an den Zähnen des Benutzers abgeformt werden, Abkühlen der Abdruckelemente (40, 50), und Einstellen der relativen Position der Oberkiefer- und der Unterkieferschale (10, 20) mittels der einstellbaren Verbindungsvorrichtung (30).

## Claims

1. An intraoral orthosis comprising a first shell (10) and a second shell (20), said shells (10, 20) being connected together by an adjustable connection device (30), said adjustable connection device (30) comprising two notched tabs (31) that are arranged respectively on either side of one of the shells(10, 20), each notched tab (31) co-operating in adjustable manner with a respective adjustment housing (32), arranged correspondingly on either side of the other of said shells (10, 20), each notched tab (31) having adjustment teeth (312)to adjust the position of the shells (10, 20) one relative to the other, each adjustment housing (32) including at least one adjustment tooth (322) adapted to co-operate with at least one respective adjustment tooth (312) of the corresponding notched tab (31), so as to define a position of the first shell (10) relative to the second shell (20),
**characterized in that** each notched tab (31)comprises locking housings (311) to lock the position of the shells (10, 20) one relative to the other, said locking housings (311) being oriented transversally relative to said adjustment teeth (312),and it that each adjustment housing (32) includes a locking system (35) comprising a locking element (35) that is movable relative to said adjustment housing (32) between a locked position, in which locking projections (351) of the locking element (35) co-operate with said locking housings (311) of said notched tab (31), and an unlocked position in which said locking projections (351) do not co-operate with said locking housings (311).

2. An orthosis according to claim 1, wherein the relative position of the two shells (10, 20) being adjustable between a pushed back position and an advanced position of the mandibular shell.

3. An orthosis according to any preceding claim, wherein the shells (10, 20) are made by molding and/or folding.

4. An orthosis according to claim 3, wherein the shells (10, 20) are made of a material that is relatively flexible, such as polypropylene or Rilsan®, making it possible to adapt each shell to the corresponding dental arch.

5. An orthosis according to any preceding claim, wherein the adjustment housings (32) are molded integrally with their respective shell (20, 10), each adjustment housing (32) being connected to said shell (20, 10) by a foldable support structure (34).

6. An orthosis according to any preceding claim, wherein said locking projections and housings (351, 311) are in the shape of complementary grooves.

7. An orthosis according to any preceding claim, wherein said locking element (35) includes at least one shoulder (355) that co-operates with said adjustment housing (32) in the unlocked position, so as to prevent said locking element from being removed from said adjustment housing.

8. An orthosis according to any preceding claim, wherein said locking element (35) includes at least one bead (356) that co-operates with said adjustment housing (32) while said locking element is moving between its locked and unlocked positions, so as to create resistance to movement and thus prevent any unwanted movement.

9. An orthosis according to any preceding claim, wherein each shell (10, 20) includes a respective imprint element (40, 50) that is made of a thermoformable material for matching the shape of the user's teeth, each imprint element (40, 50) being fastened to its respective shell (10, 20) by being molded therein.

10. An orthosis according to claim 9, wherein the imprint elements (40, 50) are made of a material that is substantially rigid at ambient temperature and substantially deformable when heated.

11. An orthosis according to claim 10, wherein the imprint elements (40, 50) are made of a material that is selected from the family of polycapronolactones (PCL), such as CAPA®.

12. An orthosis according to any one of claims 9 to 11, wherein said shells (10, 20) include fastener profiles (35, 36), such as ribs, grooves, or projections for improving the fastening of the imprint elements (40, 50) while they are being molded.

13. A method of fabricating an orthosis according to any preceding claim, said method comprising the following steps: molding the maxillary and mandibular shells (10, 20); molding the imprint elements (40, 50) in said shells (10, 20); fabricating the adjustable connection device (30), in particular by folding at least one portion of at least one shell (10, 20); and assembling the shells (10, 20) at the adjustable connection device (30).

14. A method of adjusting an orthosis fabricated in accordance with the method of claim 13, the adjustment method comprising the following steps: heating the imprint elements (40, 50) to a working temperature in which the imprint elements are deformable; putting the orthosis with its heated imprint elements (40, 50) in place in the user's mouth so as to form splints by imprinting the user's teeth in the imprint elements (40, 50); cooling said imprint elements (40, 50); and adjusting the relative position of the maxillary and mandibular shells (10, 20) by means of the adjustable connection device (30).
